Europäisches Patentamt

European Patent Office

Office européen des brevets

(11) Veröffentlichungsnummer: **0 346 718 B1**

# (12) EUROPÄISCHE PATENTSCHRIFT

(45) Veröffentlichungstag der Patentschrift: **30.12.92**

(21) Anmeldenummer: **89110121.4**

(22) Anmeldetag: **05.06.89**

(51) Int. Cl.⁵: **C07C 29/86**, C07C 31/20

(54) **Verfahren und Vorrichtung zum Abtrennen von Propylenglykol.**

(30) Priorität: **13.06.88 DE 3820040**

(43) Veröffentlichungstag der Anmeldung:
**20.12.89 Patentblatt 89/51**

(45) Bekanntmachung des Hinweises auf die
Patenterteilung:
**30.12.92 Patentblatt 92/53**

(84) Benannte Vertragsstaaten:
**AT BE CH DE ES FR GB GR IT LI LU NL SE**

(56) Entgegenhaltungen:
**EP-A- 0 254 189**
**DD-A- 218 613**

(73) Patentinhaber: **Henkel Kommanditgesellschaft
auf Aktien
Postfach 1100 Henkelstrasse 67
W-4000 Düsseldorf 1(DE)**

(72) Erfinder: **Carduck, Franz-Josef, Dr.
Landstrasse 18
W-5657 Haan(DE)**
Erfinder: **Jeromin, Lutz, Dr.
Am Bandsbusch 88
W-4010 Hilden(DE)**
Erfinder: **Göbel, Gerd, Dr.
Falkenweg 6
W-5000 Köln 40(DE)**
Erfinder: **Johannisbauer, Wilhelm, Dr.
Erich-Kästner-Strasse 26
W-4006 Erkrath 1(DE)**
Erfinder: **Fieg, Georg, Dr.
Beckhauser Strasse 9
W-4006 Erkrath 2(DE)**
Erfinder: **Fleckenstein, Theo
Pfitzner Strasse 9
W-4010 Hilden(DE)**

## Beschreibung

Die Erfindung betrifft ein Verfahren zum Abtrennen von Propylenglykol aus einem Gemisch niedrigsiedender Fettalkohole und Propylenglykol. Die Erfindung betrifft ferner eine Vorrichtung zum Durchführen dieses Verfahrens.

Unter dem Begriff "niedrigsiedender Fettalkohole" sind solche Fettalkohole gemeint, deren Siedepunkte nahe am Siedepunkt von Propylenglykol liegen. Aufgrund dieser Eigenschaft scheidet eine Rektifikation zum Abtrennen des Propylenglykols aus.

Der Erfindung liegt die Aufgabe zugrunde, ein Verfahren zum Abtrennen von Propylenglykol aus einem Gemisch niedrigsiedender Fettalkohole und Propylenglykol zu schaffen, welches wirtschaftlich ist und ein nahezu vollständiges Entfernen des Propylenglykols aus Fettalkoholen ermöglicht. Eine Lösung dieser Aufgabe wurde erst nach umfangreichen Versuchen gefunden.

Diese Aufgabe wird erfindungsgemäß bei einem Verfahren der eingangs genannten Art dadurch gelöst, daß aus dem Gemisch mittels Wasser Propylenglykol extrahiert wird. Bei der Vorrichtung zum Durchführen des Verfahrens wird diese Aufgabe erfindungsgemäß durch mindestens eine Extraktionskolonne und mindestens eine nachgeschaltete, daran angeschlossene Rektifikationskolonne zum Abtrennen von Wasser aus Propylenglykol gelöst.

Um aus dem Extrakt, der Propylenglykol-Wasser-Mischung nahezu wasserfreies Propylenglykol herzustellen, wird erfindungsgemäß vorgeschlagen, daß aus der Propylenglykol-Wasser-Mischung durch Rektifikation nahezu wasserfreies Propylenglykol abgetrennt wird. Aufgrund sehr vorteilhafter Gleichgewichtsdaten im System Wasser/Propylenglykol reichen drei bis fünf theoretische Stufen einer Rektifikationskolonne aus, um aus dem Extrakt ein Produkt zu erhalten, daß, wenn von einem Extrakt von 35 bis 45 Gew.-% Propylenglykol und 55 bis 65 Gew.-% Wasser ausgegangen wird, 97 bis 99 Gew.-% Propylenglykol enthält.

Entsprechend wird für die Vorrichtung zum Herstellen nahezu wasserfreien Propylenglykols aus einer Fettalkohol-Propylenglykol-Mischung erfindungsgemäß vorgeschlagen, daß diese Vorrichtung mindestens eine Extraktionskolonne und mindestens eine nachgeschaltete, daran angeschlossene Rektifikationskolonne aufweist.

Dieses Verfahren und diese Vorrichtung findet insbesondere Anwendung beim Aufarbeiten des bei der direkten Hydrierung von Glyceridölen anfallenden Reaktionsgemisches. Dieses Reaktionsgemisch enthält bei Hydrierumsätzen zwischen 97 bis 99% vorwiegend Fettalkohole entsprechend der C-Kettenverteilung des eingesetzten Glyceridöls und Propylenglykol entsprechend dem Anteil an gebundenem Glycerin sowie Leichtsieder. Das Ziel eines Aufarbeitungsverfahrens in diesem Zusammenhang besteht darin, propylenglykolfreie Fettalkohole sowie wasserfreies Propylenglykol herzustellen. Versuche zeigten, daß es in diesem Fall besonders vorteilhaft ist, wenn zuerst das Reaktionsgemisch in der Weise fraktioniert wird, daß Propylenglykol und niedrigsiedende Fettalkohole als Vorlauf abgetrennt werden.

Dabei wird der Hydrierablauf in die Siedeschnitte

I. Wasser/Leichtsieder

II. Vorlauf: $C_6/C_{10}$-Fettalkohole und Propylenglykol

III. Hauptlauf: $C_{12}/C_{18}$-Fettalkohole

fraktioniert. Eine Analyse einer Hauptlaufprobe zeigt spezifikationsgerechte $C_{12}/C_{18}$-Fettalkohole die z.B. für die Sulfatierung gut geeignet sind. Dies gilt nicht für den Vorlauf, der wegen des darin enthaltenen Propylenglykols weiter aufgearbeitet werden muß. Hierzu wird die oben erwähnte extraktive Aufarbeitung vorgeschlagen. Theoretisch wäre zwar eine Extraktion des gesamten Fettalkohols, nachdem Wasser/Leichtsieder abgetrennt worden sind, denkbar, aber eine solche Extraktion ist aus wirtschaftlichen Gründen unvorteilhaft.

Um eine hohe Konzentration an Propylenglykol im Extrakt zu ermöglichen und damit z.B. beim Herstellen von nahezu wasserfreiem Propylenglykol niedrige Weiterverarbeitungskosten zu erreichen, wird vorgeschlagen, daß die Extraktion des Propylenglykols in Apparaturen erfolgt, die mehrere theoretische Trennstufen aufweisen. Vorteilhaft ist auch, wenn die Extraktion der Propylenglykol-Wasser-Mischung kontinuierlich, insbesondere im Gegenstrom, durchgeführt wird.

Besonders effektiv wird die Extraktion in mindestens einer Siebboden-Extraktions-Kolonne durchgeführt.

Ferner ist vorteilhaft, wenn zum Extrahieren mindestens eine pulsierte Extraktionskolonne verwendet wird.

Um Propylenglykol von hoher Qualität hinsichtlich Farbe, Geruch und Zusammensetzung zu erreichen, wird vorgeschlagen daß das aus der Rektifikation erhaltene nahezu wasserfreie Propylenglykol mit Aktivkohle behandelt wird oder gegebenenfalls in einer weiteren Kolonne über Kopf destilliert wird. Im letzten Fall wird eine Reinheit von 99,69%, eine Säurezahl SZ kleiner 0,01, eine Verrseifungszahl VZ = 0,15 und ein Wasseranteil von 0,16% erreicht.

Bei der erfindungsgemäßen Vorrichtung ist es vorteilhaft, wenn die Extraktionskolonne mehrstufig ist.

Besonders wirtschaftlich ist es, wenn die Extraktionskolonne Einbauten, insbesondere Siebböden aufweist.

Ferner ist vorteilhaft, wenn die Extraktionskolonne eine pulsierte Kolonne ist.

Insbesondere ist eine Vorrichtung vorteilhaft, die mindestens eine weitere Rektifikationskolonne aufweist, die mit ihrem Vorlauf-Ausgang an den Eingang der Extraktionskolonne angeschlossen ist. Mit dieser weiteren Rektifikationskolonne läßt sich nämlich z.B. der Hydrierablauf in Nasser und Leichtsieder, den Vorlauf und den Hauptlauf fraktionienen. Im übrigen Teil der erfindungsgemäßen Vorrichtung wird dann nur der Vorlauf weiterverarbeitet.

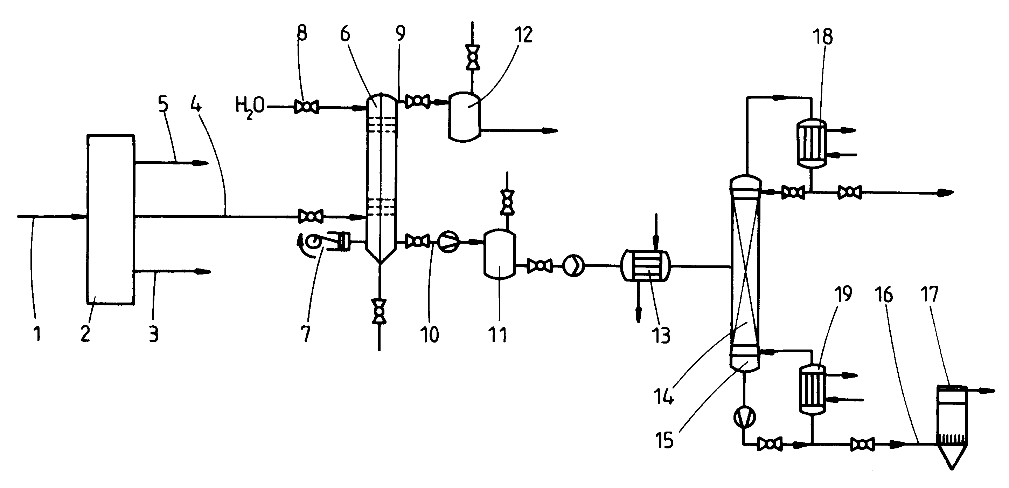

Die Erfindung wird nachstehend anhand eines Ausführungsbeispiels und der Zeichnung näher erläutert.

Die Zeichnung zeigt ein Fließbild einer erfindungsgemäßen Anlage für die Aufarbeitung des Hydrierablaufs aus der Direkthydrierung von Glyceridölen.

Eine gaschromatographische Analyse des Hydrierablaufs ergab die Werte 84 Gew.-% Fettalkohole, 0,2 Gew.-% Kohlenwasserstoffe, 10,5 Gew.-% Propylenglykol, 1 Gew.-% i- und n-Propanol sowie 2,8 Gew.-% Wasser und Spuren von Methanol und Ethanol.

Das gesamte Aufarbeitungsverfahren des Hydrierablaufs besteht aus drei Schritten:

1. Fraktionieren des Hydrierablaufs
2. Extraktion des Propylenglykols aus dem Fraktioniervorlauf im Extraktionsapparat
3. Rektifikation des Extrakts aus dem Extraktionsapparat und anschließendes Behandeln des Produkts mit Aktivkohle.

Der Hydrierablauf (1) wurde in einer Rektifikationskolonne (2) in drei Siedeschnitte fraktioniert. Die Gewichtsanteile der drei Fraktionen betrugen entsprechend 5%, 22% und 73%. Eine Analyse des Hauptlaufs (3) ergab $C_{10}$ : 0,8%, $C_{12}$ : 51%, $C_{14}$ : 21,7%, $C_{16}$ : 11,2%, $C_{18}$ : 14,9%, $C_{20}$ : 0,1%, Kohlenwasserstoffe :0,04%, Wasser : 0,06%. Wasser und Leichtsieder wurden bei (5), am Kopf der Kolonne, abgetrennt. Der Vorlauf (4) (Fettalkohole/Propylenglykol) wurde in einer pulsierenden Siebboden-Extraktionskolonne (6) extrahiert. Die Extraktionskolonne bestand aus 40 Siebböden (freier Querschnitt 14%) im Abstand von 100 mm. Die Nennweite der Kolonne betrug 100 mm. Die Pulsationseinrichtung (7) arbeitet mit einem Pulsationshub von 15 mm und einer Pulsationsfrequenz von 1,5 bis 1,6 l/s.

Der Vorlauf (4) wurde im unteren Teil, das Extraktionsmittel (8), Wasser, im oberen Teil der Kolonne (6) zugeführt. Die Durchsatze der beiden Phasen beliefen sich auf:
Vorlauf: 90 bis 100 dm³/h, Wasser: 40 bis 50 dm³/h`, wobei Wasser aufgrund umfangreicher Vorversuche als kontinuierliche Phase gewählt wurde. Als Raffinat (9) und Extrakt (10) wurden jeweils farblose Flüssigkeiten erhalten. Das Raffinat (9) weist 95 bis 97 Gew.-% an Fettalkoholen, 3 bis 4 Gew.-% an $H_2O$ und kein Propylenglykol auf.

Das Extrakt (10) setzt sich aus 35 bis 45 Gew.-% Propylenglykol, 55 bis 65 Gew.-% Wasser und Spuren erwähnter Leichtsieder zusammen.

Das Raffinat (9) wird im Behälter (12) aufgefangen.Das Extrakt aus dem Behälter (11) wird im Wärmeaustauscher (13) auf die Betriebstemperatur (ca. 100ºC) gebracht und der Rektifikationskolonne (14) zugeführt. Es wurde eine Rektifikationskolonne mit Packung (Kühni Rombopack 4 m Höhe, Durchmesser 316 mm) eingesetzt. Sie wurde mit einem Rücklaufverhältnis von 0,5 unter Normaldruck (1 bar) betrieben. Der Extraktdurchsatz belief sich auf ca. 70 kg/h, was bei dem erwähnten Rücklaufverhältnis einen Energieeinsatz im Verdampfer (19) der Kolonne von ca 40 bis 47 kW in Anspruch nahm. Die Durchsätze der beiden Kopf- und Sumpfprodukte betrugen entsprechend 42 kg/h und 28 kg/h. Die leichte gelbliche Verfärbung des Sumpfproduktes (16) (97 bis 99 Gew.-% Propylenglykol) sowie seine geringe geruchliche Belastung konnten problemlos durch anschließende Bleichung (17) mit Aktivkohle (z.B. NORIT CA1 oder BRILLONIT; Aktivkohleverbrauch: ca. 1 bis 2 g/100g) vollständig entfernt werden.

In der Zeichnung sind außerdem bei (15) der Kolonnensumpf und bei (18) ein Kondensator dargestellt.

## Bezugszeichenliste

| 1 | Hydrierablauf |
|---|---|
| 2 | Rektifikationskolonne |
| 3 | Hauptlauf |
| 4 | Vorlauf |
| 5 | Wasser und Leichtsieder |
| 6 | (Siebboden-)Extraktionskolonne |
| 7 | Pulsationseinrichtung |
| 8 | Extraktionsmittel |
| 9 | Raffinat |
| 10 | Extrakt |
| 11 | Behälter |
| 12 | Behälter |
| 13 | Wärmeaustauscher |
| 14 | Rektifikationskolonne |
| 15 | Kolonnensumpf |
| 16 | Sumpfprodukt |
| 17 | Bleichung |
| 18 | Kondensator |
| 19 | Verdampfer |

## Patentansprüche

1. Verfahren zum Abtrennen von Propylenglykol aus einem Gemisch niedrigsiedender Fettalkohole und Propylenglykol, dadurch gekennzeichnet, daß aus dem Gemisch mittels Wasser Propylenglykol extrahiert wird.

2. Verfahren nach Anspruch 1, dadurch gekennzeichnet, daß zum Herstellen nahezu wasserfreien Propylenglykols nach dem Extrahieren aus der Propylenglykol-Wasser-Mischung durch Rektifikation nahezu wasserfreies Propylenglykol abgetrennt wird.

3. Verfahren nach Anspruch 1 oder 2, wobei von dem bei der Direkthydrierung von Glyceridölen entstehenden Reaktionsgemisch ausgegangen wird, dadurch gekennzeichnet daß zuerst das Reaktionsgemisch in der Weise fraktioniert wird, daß Propylenglykol und niedrigsiedende Fettalkohole als Vorlauf abgetrennt werden.

4. Verfahren nach einem der Ansprüche 1 bis 3, dadurch gekennzeichnet, daß die Extraktion des Propylenglykols in Apparaturen erfolgt, die mehrere theoretische Trennstufen aufweisen.

5. Verfahren nach einem der Ansprüche 1 bis 4, dadurch gekennzeichnet, daß die Extraktion der Propylenglykol-Wasser-Mischung kontinuierlich, insbesondere im Gegenstrom, durchgeführt wird.

6. Verfahren nach einem der Ansprüche 1 bis 5, dadurch gekennzeichnet, daß die Extraktion in mindestens einer Siebboden-Extraktionskolonne durchgeführt wird.

7. Verfahren nach einem der Ansprüche 1 bis 6, dadurch gekennzeichnet, daß zum Extrahieren mindestens eine pulsierte Extraktionskolonne verwendet wird.

8. Verfahren nach einem der Ansprüche 2 bis 7, dadurch gekennzeichnet, daß das nahezu wasserfreie Propylenglykol mit Aktivkohle behandelt wird.

9. Vorrichtung zum Durchführen des Verfahrens nach einem der Ansprüche 2 bis 8, gekennzeichnet durch mindestens eine Extraktionskolonne (6) und mindestens eine nachgeschaltete, daran angeschlossene Rektifikationskolonne (14) zum Abtrennen von Wasser aus Propylenglykol.

10. Vorrichtung nach Anspruch 9, dadurch gekennzeichnet, daß die Extraktionskolonne (6) mehrstufig ist.

11. Vorrichtung nach Anspruch 9 oder 10, dadurch gekennzeichnet, daß die Extraktionskolonne (6) Einbauten aufweist.

12. Vorrichtung nach Anspruch 11, dadurch gekennzeichnet, daß die Extraktionskolonne (6) Siebböden aufweist.

13. Vorrichtung nach einem der Ansprüche 9 bis 12, dadurch gekennzeichnet, daß die Extraktionskolonne (6) eine pulsierte Kolonne ist.

14. Vorrichtung nach einem der Ansprüche 9 bis 13, gekennzeichnet durch mindestens eine weitere Rektifikationskolonne (2), die mit ihrem Vorlauf-Ausgang an den Eingang der Extraktionskolonne (6) angeschlossen ist.

**Claims**

1. A process for the separation of propylene glycol from a mixture of low-boiling fatty alcohols and propylene glycol, characterized in that propylene glycol is extracted from the mixture with water.

2. A process as claimed in claim 1, characterized in that, to produce substantially anhydrous propylene glycol, substantially anhydrous propylene glycol is separated off from the propylene glycol/water mixture by rectification after extraction.

3. A process as claimed in claim 1 or 2 starting out from the reaction mixture obtained in the direct hydrogenation of glyceride oils, characterized in that the reaction mixture is first fractionated so that propylene glycol and low-boiling fatty alcohols are separated off as first runnings.

4. A process as claimed in any of claims 1 to 3, characterized in that the extraction of the propylene glycol is carried out in apparatus comprising several theoretical separation stages.

5. A process as claimed in any of claims 1 to 4, characterized in that the extraction of the propylene glycol/water mixture is carried out continuously, more especially in countercurrent.

6. A process as claimed in any of claims 1 to 5, characterized in that the extraction is carried out in at least one sieve-plate extraction column.

7. A process as claimed in any of claims 1 to 6, characterized in that at least one pulsed extraction column is used for extraction.

8. A process as claimed in any of claims 2 to 7, characterized in that the substantially anhydrous propylene glycol is treated with active carbon.

9. An apparatus for carrying out the process claimed in any of claims 2 to 8, characterized by at least one extraction column (6) and at least one following rectification column (14) connected thereto for the separation of water from propylene glycol.

10. An apparatus as claimed in claim 9, characterized in that the extraction column (6) comprises several stages.

11. An apparatus as claimed in claim 9 or 10, characterized in that the extraction column (6) has fittings.

12. An apparatus as claimed in claim 11, characterized in that the extraction column (6) comprises sieve plates.

13. An apparatus as claimed in any of claims 9 to 12, characterized in that the extraction column (6) is a pulsed column.

14. An apparatus as claimed in any of claims 9 to 13, characterized by at least one other rectification column (2) which is connected at its first runnings exit to the entrance of the extraction column (6).

**Revendications**

1. Procédé de séparation du propylèneglycol à partir d'un mélange d'alcools gras à bas point d'ébullition et de propylèneglycol, caractérisé en ce que le propylèneglycol est extrait du mélange à l'aide d'eau.

2. Procédé selon la revendication 1, caractérisé en ce que pour l'obtention de propylèneglycol presque anhydre, du propylèneglycol presque anhydre est séparé après l'extraction, du mélange propylèneglycol/eau par rectification.

3. Procédé selon la revendication 1 ou la revendication 2 dans lequel on part du mélange réactionnel qui se forme lors de l'hydrogénation directe des huiles glycéridiques, caractérisé en ce qu'en premier lieu le mélange réactionnel est fractionné de manière que le propylèneglycol et les alcools gras à bas point d'ébullition soient séparés sous forme d'écoulement préliminaire.

4. Procédé selon l'une des revendications 1 à 3, caractérisé en ce que l'extraction du propylèneglycol s'effectue dans des appareillages qui possèdent plusieurs étapes théoriques de séparation.

5. Procédé selon l'une des revendications 1 à 4, caractérisé en ce que l'extraction du mélange propylèneglycol /eau est effectuée en continu, en particulier à contre-courant.

6. Procédé selon l'une des revendications 1 à 5 caractérisé en ce que l'extraction est effectuée dans au moins une colonne d'extraction à fond de tamisage.

7. Procédé selon l'une des revendications 1 à 6, caractérisé en ce que l'on utilise pour l'extraction au moins une colonne d'extraction pulsée.

8. Procédé selon l'une des revendications 2 à 7, caractérisé en ce que le propylèneglycol presque anhydre est traité par du charbon actif.

9. Dispositif pour l'exécution du procédé selon l'une des revendications 2 à 8, caractérisé par au moins une colonne d'extraction (6) et au moins une colonne de rectification (14) post-connectée et reliée à celle-ci, pour la séparation de l'eau du propylèneglycol.

10. Dispositif selon la revendication 9, caractérisé en ce que la colonne d'extraction (6) est à plusieurs étages.

11. Dispositif selon la revendication 9 ou la revendication 10, caractérisé en ce que la colonne d'extraction (6) comporte des montages installés.

12. Dispositif selon la revendication 11, caractérisé en ce que la colonne d'extraction (6) possède des fonds de tamisage.

13. Dispositif selon l'une des revendications 9 à 12, caractérisé en ce que la colonne d'extraction (6) est une colonne pulsée.

14. Dispositif selon l'une des revendications 9 à 13, caractérisé par au moins une colonne de rectification (2) supplémentaire qui est connectée par sa sortie de l'écoulement préliminaire à l'entrée de la colonne d'extraction (6).